# NOUVEAU FASCICULE DE BREVET EUROPEEN

(11) **EP 0 657 212 B2**
(45) Date de publication et mention de la décision concernant l'opposition: **31.12.2008**
(45) Mention de la délivrance du brevet: 19.09.2001
(21) Numéro de dépôt: 94203494.3
(22) Date de dépôt: 01.12.1994
(51) Int. Cl.: B01J 27/122, B01J 27/138, B01J 27/10, C07C 17/156

(54) **Utilisation d'une composition catalytique dans un procédé d'oxychloration de l'éthylène**
Verwendung einer katalytischen Zusammensetzung in einem Verfahren zur Oxychlorierung von Ethylen
Use of a catalytic composition in an ethylene oxychlorination process

(30) Priorité: 08.12.1993 BE 9301354
(43) Date de publication de la demande: 14.06.1995
(73) Titulaire: SOLVAY (Société Anonyme), 1050 Bruxelles (BE)
(72) Inventeur: Derleth, Helmut, D-3070 Nienburg (DE); Adem, Deniz, B-7133 Binche (BE); Strebelle, Michel, B-1150 Bruxelles (BE)
(74) Mandataire: Jacques, Philippe

(56) Documents cités:
- EP-A- 0 255 156
- EP-A- 0 375 202
- EP-A- 0 494 474
- EP-A1- 0 582 165
- EP-A1- 0 657 212
- US-A- 3 624 170
- US-A- 4 446 249
- US-A- 5 004 849
- ZHERNOSCK V.M. ET AL: 'Translation from Kinetika i Kataliz' vol. 12, no. 2, 1971, pages 407 - 413

## Description

La présente invention se rapporte à une utilisation d'une composition catalytique dans un procédé d'oxychloration de l'éthylène.

L'oxychloration, c'est-à-dire la chloration d'hydrocarbures par du chlorure d'hydrogène en présence d'air ou d'oxygène, constitue une réaction connue de longue date qui s'effectue habituellement en présence de catalyseurs constitués par des sels métalliques déposés sur des supports inertes tels que des alumines, des gels de silice, des oxydes mixtes ou encore des argiles ou autres supports d'origine naturelle. Industriellement, le catalyseur est utilisé le plus fréquemment en lit fluide mais peut aussi être employé en lit fixe. Comme sels métalliques, on utilise le plus souvent des halogénures tels que le chlorure de cuivre. Lorsqu'il est utilisé seul, le chlorure de cuivre présente toutefois l'inconvénient d'être relativement volatil, ce qui entraîne une chute de l'activité catalytique et du rendement de la réaction d'oxychloration, inacceptable dans les installations industrielles.

Il est bien connu d'améliorer les performances des catalyseurs d'oxychloration constitués de chlorure de cuivre sur support par ajout de chlorures de métaux alcalins, de métaux alcalino-terreux ou de terres rares (lanthanides). En particulier, des compositions catalytiques pour l'oxychloration comprenant simultanément des chlorures de cuivre, de magnésium et de métaux alcalins sur alumine ont déjà été proposées.

La demande EP-A-0255156 de SOLVAY décrit des compositions catalytiques ternaires contenant un mélange des chlorures de cuivre, de magnésium et d'un métal alcalin choisi parmi le sodium ou le lithium utilisés dans des proportions précises et la demande EP-A-0494474 décrit des compositions catalytiques comprenant des chlorures de cuivre, de magnésium, de lithium et d'au moins un chlorure de métal alcalin autre que le lithium, qui permettent d'atteindre un très bon rendement en 1,2-dichloréthane dans un procédé en lit fluide d'oxychloration de l'éthylène, en réduisant simultanément la corrosion des réacteurs en acier inoxydable, grâce, notamment à une réduction du collage et du mottage des grains de catalyseur.

La demande EP-A-0375202 envisage des compositions catalytiques ternaires à base des chlorures de cuivre, de magnésium et de potassium, contenant 30 à 90 g de cuivre, de 2 à 30 g de magnésium et de 2 à 30 g de potassium par kilo de composition catalytique, avec un rapport atomique Cu : Mg : K de 1 : 0,1-1,0 : 0,1-1,0.

Il a cependant été observé que la plupart des compositions de l'art antérieur comprenant simultanément des chlorures de cuivre, de magnésium et de métaux alcalins déposés sur alumine, provoquent, dans les réacteurs d'oxychloration de l'éthylène en lit fluidisé, le dépôt de salissures à la surface des tubes du faisceau de l'échangeur thermique disposé dans le lit fluide. Ce phénomène a été observé en particulier dans les procédés à l'oxygène, procédés dans lesquels l'oxygène est mis en oeuvre soit sous forme pure, soit sous la forme d'un mélange d'oxygène et d'azote plus riche en oxygène que l'air. Ce comportement des compositions catalytiques constitue un obstacle important à leur utilisation. En effet, il se forme peu à peu une couche de salissures de plus en plus épaisse à la surface des tubes, ce qui entraîne une dégradation progressive du transfert thermique. En outre, ce phénomène peut, à terme, induire de la corrosion. Il est dès lors indispensable de procéder à des arrêts réguliers des réacteurs pour nettoyer les tubes du faisceau de l'échangeur thermique.

Un des objectifs de la présente invention est dès lors d'obtenir, dans un procédé d'oxychloration de l'éthylène en lit fluide, un rendement en 1,2-dichloréthane élevé sans provoquer le dépôt de salissures à la surface des tubes du faisceau de l'échangeur thermique, spécialement dans les procédés à l'oxygène.

En conséquence, la présente invention concerne une utilisation telle que définie dans la revendication 1.

De manière surprenante, on a maintenant observé que des compositions catalytiques contenant des chlorures de cuivre, de magnésium et de potassium, dans les quantités spécifiées ne provoquent pas, à la surface des tubes du faisceau de l'échangeur thermique disposé dans le lit fluide, le dépôt de salissures observé avec les compositions de l'art antérieur, tout en permettant d'atteindre, en oxychloration de l'éthylène en 1,2-dichloréthane, une sélectivité en 1,2-dichloréthane par rapport à l'éthylène converti et un rendement en 1,2-dichloréthane par rapport au chlorure d'hydrogène mis en oeuvre similaires, voire meilleurs, à ceux obtenus avec les compositions de l'art antérieur.

Les compositions catalytiques utilisées selon l'invention contiennent au moins 30 g de cuivre par kilo de composition catalytique, de manière préférée au moins 40 g par kilo et, de manière particulièrement préférée, au moins 50 g par kilo. Elles contiennent au plus 90 g de cuivre par kilo de composition catalytique. Celles en contenant au plus 80 g par kilo apparaissent avantageuses. Celles en contenant au plus 70 g par kilo apparaissent particulièrement avantageuses.

Les compositions catalytiques utilisées selon l'invention contiennent au moins 12 g de magnésium par kilo de composition catalytique, et, de manière particulièrement préférée, au moins 15 g par kilo. Elles contiennent au plus 30 g de magnésium par kilo de composition catalytique. Celles en contenant au plus 25 g par kilo apparaissent avantageuses. Celles en contenant au plus 20 g par kilo apparaissent particulièrement avantageuses.

Les compositions catalytiques utilisées selon l'invention contiennent au moins 0,1 g de potassium par kilo de composition catalytique, de manière préférée au moins 0,5 g par kilo et, de manière particulièrement préférée, au moins 1 g par kilo. Elles contiennent au plus 10 g de potassium par kilo de composition catalytique. Celles en contenant au plus 9 g par kilo apparaissent avantageuses. Celles en contenant au plus 6 g par kilo apparaissent particulièrement avantageuses.

De bons résultats en oxychloration de l'éthylène ont été obtenus par l'utilisation de compositions catalytiques contenant de 40 à 80 g de cuivre, de 12 à 25 g de magnésium et de 0,5 à 9 g de potassium par kilo de composition catalytique.

Dans les compositions selon l'invention, le rapport atomique Mg/Cu est de préférence au moins de 0,3 et, de manière particulièrement préférée, au moins de 0,5. Avantageusement, ce rapport ne dépasse pas 1,5. Très avantageusement, il ne dépasse pas 1,0.

Le rapport atomique K/Cu est de préférence au moins de 0,01 et, de manière particulièrement préférée, au moins de 0,025. Avantageusement, ce rapport ne dépasse pas 0,30. Très avantageusement, il ne dépasse pas 0,25.

Le rapport atomique K/Mg est de préférence au moins de 0,01 et, de manière particulièrement préférée, au moins de 0,025. Avantageusement, ce rapport ne dépasse pas 0,8. Très avantageusement, il ne dépasse pas 0,5.

De très bons résultats en oxychloration de l'éthylène ont été obtenus avec des compositions présentant des rapports atomiques Cu : Mg : K de 1 : 0,5-1,0 : 0,025-0,25.

L'alumine mise en oeuvre comme support dans les compositions catalytiques utilisées selon l'invention peut être de toute origine et être obtenue selon tout procédé connu; on utilise habituellement des alumines de type êta ou gamma. De bons résultats ont été obtenus avec une alumine gamma. L'alumine mise en oeuvre dans les compositions catalytiques utilisées selon l'invention présente généralement un diamètre moyen des particules compris entre 10 et 200 *µ*m et de préférence un diamètre moyen compris entre 20 et 120 *µ*m. Sa surface spécifique, mesurée suivant la méthode B.E.T. est généralement comprise entre 50 m²/g et 250 m²/g. De bons résultats en oxychloration de l'éthylène ont été obtenus avec une alumine présentant une surface spécifique de 100 m²/g à 210 m²/g. Enfin, le volume poreux des alumines habituellement utilisées se situe entre 0,1 et 1 cm³/g. De préférence, le volume poreux est compris entre 0,2 et 0,8 cm³/g et de bons résultats en oxychloration de l'éthylène ont été obtenus avec une alumine présentant un volume poreux de 0,3 à 0,6 cm³/g.

Le mode d'obtention des compositions catalytiques utilisées selon l'invention n'est pas critique. Les chlorures métalliques peuvent être introduits dans la composition catalytique soit directement sous forme de chlorures, par exemple par imprégnation du support à l'aide d'une solution renfermant un mélange de ces sels, soit sous forme d'autres composés des métaux, tels que les oxydes, les hydroxydes, les nitrates ou tout autre composé capable d'être transformé en chlorure dans les conditions dans lesquelles sont menées les réactions d'oxychloration. La préparation des compositions catalytiques peut notamment être réalisée dans un tambour rotatif ou dans un lit fluidisé, par imprégnation de l'alumine avec une solution des chlorures métalliques, en une ou en plusieurs passes, en présence ou non d'additifs tels que des acides, par exemple l'acide chlorhydrique.

Un mode d'obtention ayant donné de bons résultats consiste à imprégner une alumine avec une solution aqueuse renfermant les quantités adéquates des chlorures de cuivre, de magnésium et de potassium dans lequel on évite l'apparition d'une phase liquide non adsorbée par le solide en limitant le volume de la solution imprégnante à 70 à 100 % du volume poreux de la quantité d'alumine mise en oeuvre. L'alumine imprégnée est ensuite séchée avant d'être introduite dans le réacteur d'oxychloration proprement dit.

Les compositions catalytiques finales présentent généralement une surface spécifique B.E.T. comprise entre 25 m²/g et 200 m²/g et de préférence entre 50 et 150 m²/g. De bons résultats en oxychloration de l'éthylène ont été obtenus avec des compositions catalytiques présentant une surface spécifique B.E.T. de 80 à 140 m²/g.

L'utilisation selon l'invention convient pour les procédés d'oxychloration à l'air ou à l'oxygène. Elle est particulièrement bien adaptée au procédé à l'oxygène, utilisant de l'oxygène pur ou un mélange oxygène/azote plus riche en oxygène que l'air.

Lorsque l'on opère avec un catalyseur disposé en lit fluidisé, la température à laquelle s'effectue la réaction d'oxychloration se situe habituellement entre 200 et 300 °C. De préférence, cette température est comprise entre 220 et 280 °C. Enfin, des bons résultats ont été obtenus à une température située aux environs de 230 - 270 °C.

La pression à laquelle est effectuée la réaction d'oxychloration n'est pas critique en elle-même. Habituellement, on opère avec des pressions comprises entre 0,1 et 1 MPa et de préférence avec des pressions comprises entre 0,1 et 0,8 MPa. La vitesse de fluidisation des compositions catalytiques n'est pas critique en elle-même et dépend essentiellement de la granulométrie du catalyseur et des dimensions de l'appareillage. Généralement, on opère avec des vitesses comprises entre 5 et 100 cm/s. Enfin, le rapport des réactifs mis en oeuvre est le même que celui généralement utilisé dans les procédés antérieurs. D'habitude, on opère avec un léger excès d'éthylène par rapport à la quantité stoechiométrique nécessaire pour réagir avec l'HCl mis en oeuvre. Toutefois, les compositions catalytiques utilisées selon l'invention permettent indifféremment de travailler avec des excès importants d'éthylène ou au voisinage de la stoechiométrie, voire même en excès d'HCl.

L'invention se trouve plus amplement illustrée par les exemples suivants. Les exemples annotés (c) se rapportent à des exemples donnés à titre comparatif.

### Exemples 1 à 9

Un catalyseur a été préparé au départ d'une alumine gamma présentant une surface spécifique de 186 m²/g, un volume poreux de 0,38 cm³/g, un poids spécifique (mesuré par écoulement libre) de 0,75 kg/dm³ et un diamètre moyen des particules de 50 *µ*m. A environ 800 g de cette alumine a été ajoutée une solution aqueuse d'imprégnation comprenant à l'état dissous du CuCl₂.2H₂O, du MgCl₂.6H₂O et du KCl en quantités adéquates pour obtenir, après séchage à 150 °C, environ 1 kg de catalyseur présentant, calculée sous forme métallique par rapport au poids total du catalyseur, une teneur en cuivre de 60 g/kg, une teneur en magnésium de 18 g/kg et une teneur en potassium de 1,3 g/kg. Exprimée en rapport atomique, la proportion entre les différents métaux Cu : Mg : K est de 1 : 0,80 : 0,035.

Les catalyseurs mis en oeuvre dans les exemples 2 à 9 ont été préparés de la même manière que le catalyseur de l'exemple 1, au départ de la même alumine imprégnée par une solution aqueuse renfermant CuCl₂.2H₂O, MgCl₂.6H₂O et KCl, LiCl ou NaCl en quantités et proportions adéquates. Les teneurs en métaux dans ces différents catalyseurs sont rassemblées au tableau I.

Ces 9 catalyseurs ont été testés en oxychloration de l'éthylène dans un réacteur micro-pilote en lit fluide, contenant 225 cm³ de catalyseur. Le catalyseur est fluidisé au moyen des gaz réactifs introduits par le bas au travers d'un filtre métallique fritté. Les conditions opératoires dans lesquelles les tests ont été réalisés sont les suivantes :
- rapport 2C₂H₄/HCl = 1,07 mol/mol
- rapport 4O₂/HCl = 1,35 mol/mol
- vitesse des gaz : 10 cm/s (par rapport au réacteur vide à pression et température d'essai)
- température : 260 °C
- pression : 0,6 MPa
- temps de séjour : 5 s.

Les produits de réaction quittant le réacteur ont été détendus jusqu'à la pression atmosphérique par une vanne de régulation de pression du réacteur et refroidis dans un piège maintenu à - 20 °C. Les gaz non condensés ont été lavés dans un scrubber à eau avant de balayer une ampoule de prélèvement. Le bilan des produits formés a été effectué au départ d'analyses chromatographiques des produits liquide et gazeux recueillis et du titrage de l'acidité de la solution aqueuse recueillie au pied du scrubber. Le rendement en 1,2-dichloréthane (rapport molaire entre le DCEa formé et l'HCl mis en oeuvre) et la sélectivité en DCEa (rapport molaire entre le DCEa formé et l'éthylène converti) sont présentés au tableau I.

Le dépôt de salissures provoqué par les différents catalyseurs a été mesuré dans un réacteur micro-pilote similaire au réacteur décrit ci-avant mais muni en outre d'un tube en forme de doigt de gant, plongeant verticalement dans le lit fluide. Ce tube en doigt de gant comporte une double paroi dans laquelle circule une huile maintenue à une température inférieure à la température à laquelle est menée la réaction. Le dépôt de salissures est déterminé visuellement par examen de la surface externe de ce tube en doigt de gant après 20 heures de fonctionnement du réacteur dans les conditions suivantes :
- rapport 2C₂H₄/HCl = 1,07 mol/mol
- rapport 4O₂/HCl = 1,12
- vitesse des gaz : 2,5 cm/s
- température dans le lit fluide : 270 °C
- température à la surface externe du tube en doigt de gant : 180 °C.
- pression : 0,7 MPa
- temps de séjour : 12 s.

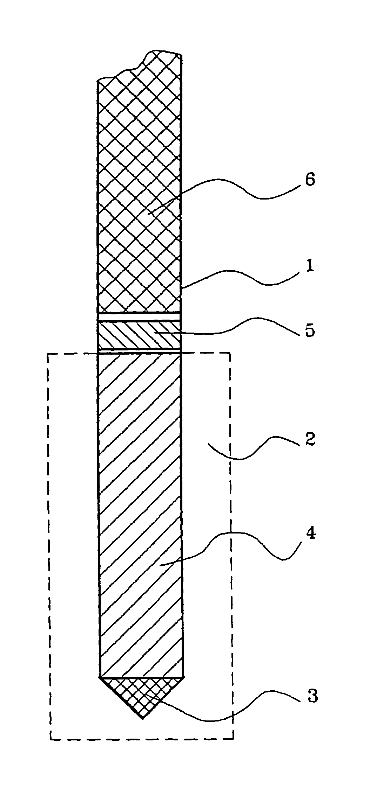

Dans ces conditions, les résultats obtenus reflètent le comportement des catalyseurs observé après quelques mois de fonctionnement dans un réacteur industriel. Une cote chiffrée est attribuée aux catalyseurs, en fonction de l'aspect des salissures et de l'endroit auquel elles apparaissent à la surface externe du tube en doigt de gant. A la figure unique, on a schématisé le tube en doigt de gant (1) plongeant dans le lit fluide (2). Le tube comporte 4 zones distinctes : une pointe conique (3), une surface cylindrique (4) plongeant dans le lit fluide (2), une interface (5), située juste au-dessus du lit fluide et une surface cylindrique (6), située hors du lit fluide, au-dessus de l'interface (5). La présence d'un voile, c'est-à-dire d'une fine pellicule adhérente qui ne comprend pas de particules de catalyseur, à la pointe (3) ou à la surface (4) plongeant dans le lit fluide vaut 1 point. La présence d'une croûte, c'est-à-dire d'un dépôt plus épais comprenant des particules de catalyseur adhérant à la surface du tube, vaut 2 points à la pointe (3) et à la surface (4) et 1 point à l'interface (5). Sur la zone de la surface (6) située hors du lit, seule la présence d'agrégats de particules de catalyseurs a parfois été observée et est comptée pour 1 point. La présence sur n'importe quelle zone de la surface du tube de particules de catalyseur non adhérentes n'est pas prise en compte. Une cote de 0 sera donc attribuée à un catalyseur ne provoquant aucun dépôt de salissures lors du test, alors qu'un catalyseur provoquant une apparition importante de salissures, mise en évidence, par exemple, par la présence de croûtes à la pointe (3) (2 points), à la surface (4) (2 points) et à l'interface (5) (1 point) se verra attribuer une cote de 5.

Les résultats obtenus sont rassemblés au tableau I, qui reprend les compositions des différents catalyseurs testés, les résultats obtenus en oxychloration de l'éthylène ainsi que les mesures de dépôt de salissures.

Les compositions catalytiques des exemples 4 à 9 de comparaison, donnent un bon rendement en 1,2-dichlor-éthane par rapport à l'HCl et une bonne sélectivité de l'éthylène en 1,2-dichloréthane mais provoquent le dépôt de salissures à la surface du tube en doigt de gant. Par contre, les exemples 1 à 3 démontrent que les compositions utilisées selon l'invention ne provoquent aucun dépôt de salissures, tout en procurant une sélectivité et un rendement en 1,2-dichloréthane très élevés.

**Tableau I**

| N° Ex. | Composition | | | | | | Rendement en DCEa par rapport à HCl (%mol) | Sélectivité en DCEa par rapport à l'éthylène converti (%mol) | Dépôt de salissures (voir texte) |
|---|---|---|---|---|---|---|---|---|---|
| | teneur poids (g/kg) | | | proportions atomiques | | | | | |
| | Cu | Mg | Alc | Cu | Mg | Alc | | | |
| 1 | 60 | 18 | 1,3K | 1 | 0,80 | 0,035K | 98,2 | 95,4 | 0 |
| 2 | 59 | 17 | 4,7K | 1 | 0,75 | 0,13K | 98,2 | 96,3 | 0 |
| 3 | 59 | 17 | 8,5K | 1 | 0,75 | 0,23K | 98,2 | 96,7 | 0 |
| 4(c) | 60 | 17 | 2.1Li | 1 | 0,74 | 0,33Li | 97,5 | 94,9 | 3 |
| 5(c) | 56 | 17 | 3,1Li | 1 | 0,79 | 0,51Li | 98,2 | 96,7 | 3 |
| 6(c) | 60 | 18 | 2,1Na | 1 | 0,80 | 0,10Na | 97,9 | 94,4 | 1 |
| 7(c) | 60 | 17 | 4,4Na | 1 | 0,74 | 0,21Na | 98,2 | 96,0 | 4 |
| 8(c) | 58 | 17 | 11K | 1 | 0,77 | 0,31K | 98,2 | 96,8 | 1 |
| 9(c) | 60 | 17 | 17K | 1 | 0,74 | 0,47K | 97,9 | 97,2 | 1 |

## Revendications

1. Utilisation d'une composition catalytique consistant en des chlorures de cuivre, de magnésium et de potassium déposés sur une alumine, **caractérisée en ce que** la composition catalytique contient de 30 à 90 g de cuivre, de 12 à 30 g de magnésium et de 0,1 à 10 g de potassium, exprimés sous forme métallique, par kilo de composition catalytique dans un procédé en lit fluide d'oxychloration de l'éthylène afin d'éviter le dépôt de salissures à la surface des tubes du faisceau de l'échangeur thermique disposé dans le lit fluide.

2. Utilisation selon la revendication 1, d'une composition catalytique contenant de 40 à 80 g de cuivre, de 12 à 25 g de magnésium et de 0,5 à 9 g de potassium, exprimés sous forme métallique, par kilo de composition catalytique.

3. Utilisation selon la revendication 1 ou 2, d'une composition catalytique dans laquelle le rapport atomique K/Cu est de 0,01 à 0,30.

4. Utilisation selon la revendication 3, d'une composition catalytique dans laquelle le rapport atomique K/Cu est de 0,025 à 0,25.

5. Utilisation selon l'une quelconque des revendications 1 à 4, d'une composition catalytique dans laquelle le rapport atomique K/Mg est de 0,01 à 0,8.

6. Utilisation selon l'une quelconque des revendications 1 à 5, d'une composition catalytique dans laquelle les rapports atomiques Cu : Mg : K sont de 1 : 0,5-1,0 : 0,025-0,25.

7. Utilisation selon l'une quelconque des reventications 1 à 6, d'une composition catalytique dans laquelle l'alumine présente une surface spécifique, mesurée suivant la méthode B.E.T. comprise entre 50 m²/g et 250 m²/g.

## Claims

1. Use of a catalytic composition consisting in copper chloride, magnesium chloride and potassium chloride deposited on an alumina, **characterized in that** the catalytic composition contains from 30 to 90 g of copper, from 12 to 30 g of magnesium and from 0.1 to 10 g of potassium, expressed as metal, per kilo of catalytic composition in a fluid bed ethylene oxychlorination process in order to avoid the deposition of soiling material on the surface of the tubes of the heat exchanger located in the fluid bed.

2. Use according to Claim 1, of a catalytic composition containing from 40 to 80 g of copper, from 12 to 25 g of magnesium and from 0.5 to 9 g of potassium, expressed as metal, per kilo of catalytic composition.

3. Use according to Claim 1 or 2, of a catalytic composition in which the K/Cu atomic ratio is from 0.01 to 0.30.

4. Use according to Claim 3, of a catalytic composition in which the K/Cu atomic ratio is from 0.025 to 0.25.

5. Use according to any of Claims 1 to 4, of a catalytic composition in which the K/Mg atomic ratio is from 0.01 to 0.8.

6. Use according to any one of Claims 1 to 5, of a catalytic composition in which the Cu : Mg : K atomic ratios are 1 : 0.5-1.0 : 0.025-0.025.

7. Use according to any one of Claims 1 to 6, of a catalytic composition in which the alumina has a specific surface, measured according to the B.E.T. method, of between 50 m²/g and 250 m²/g.

## Patentansprüche

1. Verwendung einer katalytischen Zusammensetzung, die aus Kupfer-, Magnesium- und Kaliumchloriden besteht, die auf einem Aluminiumoxid abgeschieden sind, **dadurch gekennzeichnet, dass** die katalytische Zusammensetzung, ausgedrückt in metallischer Form, 30 bis 90 g Kupfer, 12 bis 30 g magnesium und 0,1 bis 10 g Kalium pro Kilo katalytische Zusammensetzung enthält, in einem Fliessbett Verfahren zur Oxychlorierung von Ethylen, um die Ablagerung von Verunreinigungen auf der Oberfläche des Rohrbündels des Wärmetauschers, der in dem Fliessbett angeordnet ist, zu vermeiden.

2. Verwendung gemäß Anspruch 1, einer katalytischen Zusammensetzung die, augedrückt in metallischer Form, 40 bis 80 g Kupfer, 12 bis 25 g Magnesium und 0,5 bis 9 g Kalium pro Kilo katalytische Zusammensetzung enthält.

3. Verwendung gemäß Anspruch 1 oder 2, einer katalytischen Zusammensetzung in der das Atomverhältnis K/Cu 0,01 bis 0,30 beträgt.

4. Verwendung gemäß Anspruch 3, einer katalytischen Zusammensetzung in der das Atomverhältnis K/Cu 0,025 bis 0,25 beträgt.

5. Verwendung gemäß einem der Ansprüche 1 bis 4, einer katalytischen Zusammensetzung in der das Atomverhältnis K/Mg 0,01 bis 0,8 beträgt.

6. Verwendung gemäß einem der Ansprüche 1 bis 5, einer katalytischen Zusammensetzung in der die Atomverhältnisse Cu : Mg : K 1 : 0,5 - 1,0 : 0,025 - 0,25 betragen.

7. Verwendung gemäß einem der Ansprüche 1 bis 6, einer katalytischen Zusammensetzung in der das Aluminiumoxid eine spezifische Oberfläche, gemessen gemäß dem B.E.T-Verfahren, zwischen 50 m²/g und 250 m²/g aufweist.
